# EUROPEAN PATENT APPLICATION

(11) **EP 2 184 013 A1**
(43) Date of publication of application: **12.05.2010**
(21) Application number: 09386028.6
(22) Date of filing: 06.11.2009
(51) Int. Cl.: A61B 7/02

(54) **Stethoscope diaphragm cover (Stethoscope protection)**

(30) Priority: 06.11.2008 GR 20080100717
(71) Applicant: Goutou, Thaleia-Maria, Sparti (GR)
(72) Inventor: Goutou, Thaleia-Maria, Sparti (GR)

(57) **Abstract**

The stethoscope diaphragm cover is a thin plastic membrane (2) or any other proportional material, and a handle like edge, which is an extension of the membrane (1), which is the point from where one can manipulate and remove it by hand. The stethoscope diaphragm cover is placed on the stethoscope's diaphragm each time before using it for reasons of protection and hygienic towards the patient (examined), protecting him from the transfer of contagious or non, infections or any other elements from one surface to another.

The stethoscope diaphragm cover is disposable as it is for one unique use and is addressed to all doctors, veterinarians and all professions that use stethoscopes.

## Description

The stethoscope diaphragm cover is a thin plastic membrane or any other proportional material, which is placed on the diaphragm of the stethoscope and stays in contact with it by a static way without the use of any kind of glue or any kind of adhesive material until it is removed by hand.
Thanks to it's absolute thin surface it does not bother the skin or the surface in which it comes into contact in any way.

The purpose of the stethoscope diaphragm cover is that it is disposable, meaning that it is for one unique use as to be placed each time, before every use of the stethoscope, as to not transfer contagious or non, infections, or any other elements to the child or adult patient under examination.
The stethoscope diaphragm cover comes in different sizes as to fit on all types and sizes of all existing stethoscopes, pediatric, adult, veterinary or wherever else a stethoscope can be used. The product is constituted of a plastic membrane (2) and a handle like edge, which is an extension of the membrane (1), which is the point from where one can manipulate and remove it by hand.

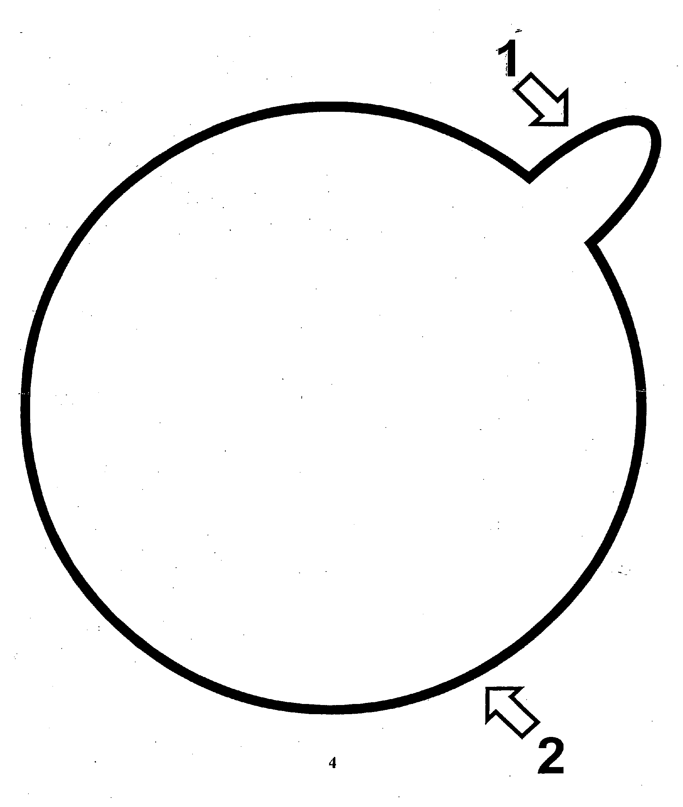

## Claims

1. The stethoscope diaphragm cover is constituted of a plastic membrane (2) and a handle like edge which is an extension of the membrane (1) which is the point from where once can manipulate and remove it by hand.

2. The stethoscope diaphragm cover according to the claim 1 is an extremely thin plastic membrane or any other
proportional material in order to prevent the transfer of skin elements infectious, contagious or not from one patient to the other.
